# EUROPÄISCHE PATENTSCHRIFT

(11) **EP 0 317 503 B1**
(45) Veröffentlichungstag und Bekanntmachung des Hinweises auf die Patenterteilung: **15.06.1994**
(21) Anmeldenummer: 88810747.1
(22) Anmeldetag: 03.11.1988
(51) Int. Cl.: A61B 17/16, A61B 10/00

(54) **Vorrichtung mit einem zum Herausschneiden eines Knorpel- oder Knochenzapfens dienenden, mit einer Antriebswelle drehfest verbundenen Hohlfräser**
Device with a hollow milling cutter linked in rotation to a primary shaft, used to draw a sample of cartilage or bone
Dispositif avec une fraise creuse liée en rotation à un axe de transmission, servant à prélever un échantillon de cartilage ou d'os

(30) Priorität: 18.11.1987 CH 4484/87
(43) Veröffentlichungstag der Anmeldung: 24.05.1989
(73) Patentinhaber: INSTITUT STRAUMANN AG, CH-4437 Waldenburg (CH)
(72) Erfinder: Burkhardt, Rolf, Prof. Dr., D-8000 München 60 (DE); Meier, Raymond Aloysius, CH-4436 Liedertswil (CH)
(74) Vertreter: Ullrich, Gerhard, Dr.

(56) Entgegenhaltungen:
- EP-A- 0 241 240
- DE-A- 2 943 162
- FR-A- 950 126
- GB-A- 2 177 307
- US-A- 4 649 918

## Beschreibung

Zum Entnehmen einer Knorpel- oder Knochenbiopsie werden üblicherweise drei Instrumente benötigt: Mit einem Hohlfräser wird Zuerst eine hohlzylindrische Bohrung angebracht. Der dadurch gebildete Knorpel oder Knochenzylinder wird dann am Zylindergrund abgeschnitten und nachher mit einer Zange oder Pinzette aus dem Knorpel- bzw. Knochen herausgeholt. Dieses Verfahren ist nicht ausgesprochen einfach, es wird jedoch bis jetzt so durchgeführt, da keine Vorrichtungen vorhanden sind, die ein einfacheres Arbeiten ermöglichen. Mit der vorliegenden erfindungsgemässen Vorrichtung lässt sich nun das Entnehmen einer Knorpel- oder Knochenbiopsie wesentlich vereinfachen, was für den Arzt und den Patienten deswegen von Vorteil ist, weil einerseits die Operationsdauer wesentlich verkürzt wird und andererseits die Biopsie schonender behandelt werden kann.

Diese neue Vorrichtung weist einen zum Herausschneiden eines Knorpel- oder Knochenzapfens dienenden, mit einer Antriebswelle drehfest verbundenen Hohlfräser auf, in dessen Innerem sich eine relativ zum Fräser längsverschiebbare, als Extraktor dienende Spannzange befindet. Aus der GB-A 2 177 307 ist nun bereits eine Vorrichtung zum Herausschneiden einer Gewebebiopsie bekannt, die einen Hohlschneider aufweist, in dessen Innerem sich eine relativ zu ihm längs verschiebbare Spannzange befindet. Dieses bekannte Instrument dient nicht nur zum Herausschneiden einer Gewebeprobe, sondern nachher auch als Aufbewahrungsort für die herausgeschnittene Probe, d.h. es wird das ganze Instrument mit der darin eingeschlossenen Probe ins Untersuchungslaboratorium gesandt, wo die Probe dann mit irgendwelchen Werkzeugen aus dem Instrument entnommen wird. Im Unterschied dazu handelt es sich beim Instrument nach der vorliegenden Erfindung um ein solches, bei welchem die Probe sofort nach dem Herausschneiden aus der Spannzange entfernt werden soll. Zu diesem Zweck ist das Instrument dadurch gekennzeichnet, dass innerhalb dieser Spannzange ein längsverschiebbarer Ausstosser angeordnet ist, und dass die Aussenwandung der Spannzange an der Innenwand der Bohrung des Hohlfräsers anliegt, dass sich die Hohlfräserwandung gegen das freie Fräserende hin nach innen so verdickt, dass einerseits an der Fräsenmündung die lichte Weite des Fräsers der lichten Weite der Spannzange in der zurückgezogenen Stellung entspricht, und andererseits die freien Enden der Spannzange sich beim nach-vorne-Verschieben einander nähern und dadurch die Grösse der Zangenöffnung verkleinern.

Nachfolgend wird anhand der beiliegenden Zeichnung ein Ausführungsbeispiel der Erfindung beschrieben. In der Zeichnung zeigt:
die Figur 1 das Ausführungsbeispiel zusammengebaut mit einem elektrischen Antrieb, teilweise in Seitenansicht und teilweise im Längsschnitt,
die Figur 2 einen Ausschnitt aus der Figur 1 in grösserem Massstab und
die Figur 3 einen Ausschnitt aus der Figur 2 in grösserem Massstab.

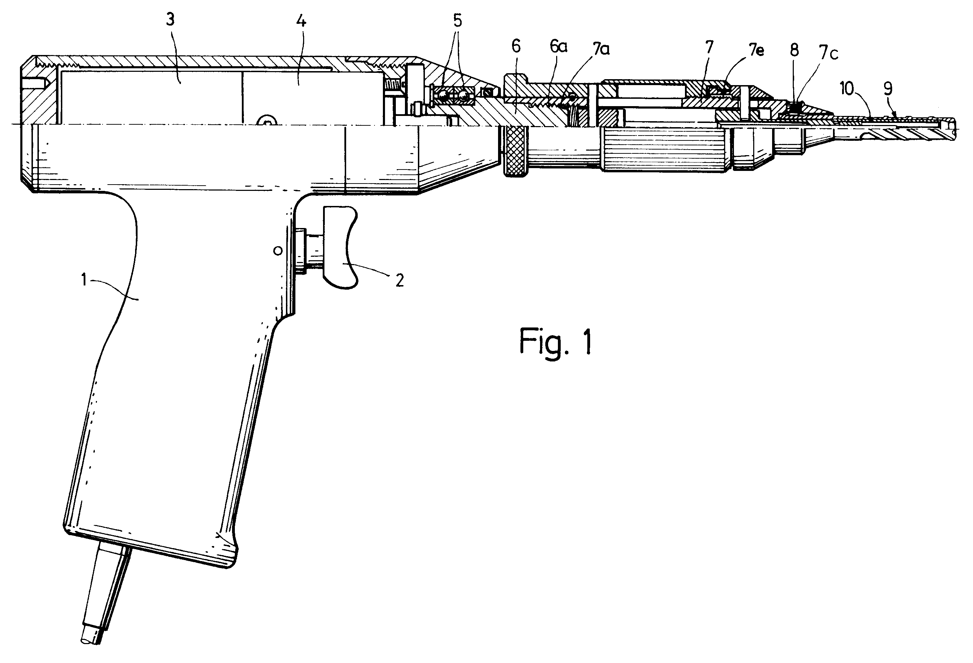

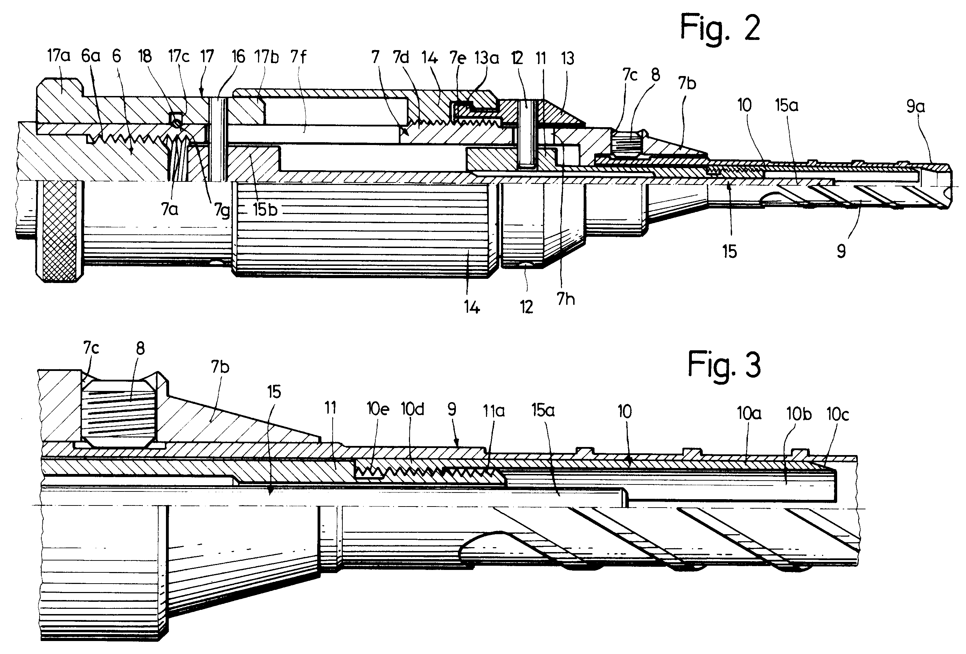

Die linke Hälfte der in der Figur 1 dargestellten Maschine zeigt eine an sich handelsübliche elektrische Bohrmaschine mit einem Handgriff 1, einem durch einen Drücker 2 ein- und ausschaltbaren Elektromotor 3, einem Untersetzungsgetriebe 4 und einer in Kugellagern 5 gelagerten Antriebswelle 6. Diese Welle besitzt an ihrem freien Ende ein Gewinde 6a, auf welchem der mit einem entsprechenden Innengewinde 7a versehene hohlzylindrische Fräserhalter 7 aufgeschraubt ist und der an seinem vordern, also dem dem Gewinde abgewandten Ende eine konische Verjüngung 7b aufweist. Direkt hinter der Verjüngung befindet sich eine radiale Gewindebohrung 7c, in welcher eine Imbus-Schraube 8 sitzt, welche den mit einer für den Angriff dieser Schraube dienenden Vertiefung versehenen, in den Fräserhalter eingesteckten, rechtsfräsenden Hohlfräser 9 lösbar festhält. Dieser Hohlfräser 9 unterscheidet sich von andern Hohlfräsern, die für das Entnehmen von Knorpel- oder Knochenbiopsien verwendet werden, höchstens dadurch, dass sich seine Wandung gegen das freie Fräserende 9a hin nach innen etwas verdickt. Wie aus den Figuren 2 und 3 besonders gut ersichtlich ist, liegt an der Innenwand dieses Hohlfräsers 9 eine Spannzange 10 an, die am freien Ende 10a durch vorteilhafterweise drei Längsschlitze in drei Lamellen 10b unterteilt ist, wobei die Lamellenenden je eine äussere Abschrägung 10c aufweisen. Am hinteren Ende ist die Spannzange 10 mit einem Innenwulst 10d versehen, der mit einem Innengewinde 10e auf dem Aussengewinde 11a des zylindrischen Zangenhalters 11 aufgeschraubt ist. Dieser Zangenhalter 11 ist durch zwei radial verlaufende Stifte 12 fest mit einer aussen auf dem Fräserhalter 7 angeordneten Schiebehülse 13 verbunden, wobei zwei Schlitze 7h dazu dienen, die axiale Verschiebung von Zangenhalter, Stift und Schiebehülse beidseits zu begrenzen und zwar so, dass in der vordern Endstellung die Zangenöffnung durch das verdickte Ende 9a des Fräsers 9 geschlossen wird, während es in der hinteren Stellung so geöffnet ist, dass die lichte Zangenweite der Spannzange der lichten Weite der Fräseröffnung entspricht.

Falls in die Vorrichtung ein Hohl-Fräser anderer Länge eingesetzt wird, was nach dem Lösen der Imbus-Schraube 8 ohne weiteres möglich ist, ist auch eine der Länge des neuen Fräsers angepasste Spannzange auf den Spannzangenhalter aufzuschrauben.

Der Fräserhalter 7 weist hinter dem Schlitz 7h eine kleine, mit einem Linksgewinde 7e versehene Verdickung 7d auf. Auf diesem Gewinde ist eine vor- und rückwärts verschraubbare Rändelmutter 14 angeordnet, mit welcher die Schiebehülse 13 durch einen Haken 13a zwar frei drehbar, aber axial unverschiebbar verhängt ist. So ist es möglich, durch Drehen der Rändelmutter 14 die Spannzange 10 vor- und rückwärts zu schieben.

Innerhalb der Spannzange 10 ist ein längsverschiebbarer Ausstosser 15 angeordnet. Dieser besteht im wesentlichen aus einem verhältnismässig dünnen Stift, der am vorderen Teil 15a im vorderen Ende des Spannzangenhalters 11 geführt ist und dessen hinteres Ende eine in der Bohrung des Fräserhalters 7 geführte Verdickung 15b aufweist, die mittels eines durchgehenden Stiftes 16 mit einer Ausstosser-Hülse 17 fest verbunden ist, deren hinteres Ende 17a auf dem Fräserhalter 7 verschiebbar aufliegt, während ihr vorderes Ende 17b durch die Rändelmutter 14 überdeckt wird. Schlitze 7f, von denen in der Zeichnung nur einer dargestellt ist, begrenzen den Verschiebeweg des Ausstossers 15 beidseits.

Ein Federring 18, der beim Aufspreizen in die U-förmige Nut 17c in der Hülse 17 ausweichen kann, bildet zusammen mit einer im Fräserhalter 7 angebrachten Nut 7g eine federnde Rast für die Ausstosserhülse, wodurch verhindert wird, dass sich diese Hülse und mit ihr der Ausstosser unbeabsichtigt nach vorne verschieben kann.

Aus dem Vorstehenden ergibt sich ohne weiteres die Funktionsweise:
Zum Entnehmen einer Knorpel- oder Knochenbiopsie wird mit dem Hohlfräser eine hohlzylindrische Bohrung im Knorpel bzw. im Knochen angebracht. Sobald die Bohrung die gewünschte oder benötigte Tiefe erreicht hat, wird der Antrieb des Motors durch Loslassen des den Schalter betätigenden Drückers 2 abgestellt und es wird mit der anderen Hand gleichzeitig die Rändelmutter 14 festgehalten. Da sich die Antriebswelle 6 und der mit ihr fest verbundene Fräserhalter 7 noch etwas drehen wird, wird die Rändelmutter 14 nach vorne geschraubt, wodurch sich auch die Schiebehülse 13 und die durch den Stift 12 mit ihr starr verbundene Spannzange 10/11 nach vorne verschieben. Infolge der innern Verdickung des Fräserendes 9a wird die Spannzange 10 geschlossen und hält den ausgefrästen Zapfen fest, sodass er beim Herausziehen des Fräsers aus der Bohrung abgebrochen und mitgenommen wird, wobei das Abbrechen auch dadurch erfolgen kann, dass sich Fräser und Spannzange bei geschlossener Zange noch etwas drehen. Alsdann wird die Rändelmutter von Hand zurückgedreht, wodurch die Spannzange zurückgezogen wird und den herausgefrästen Knorpel- bzw. Knochenzapfen freigibt. Durch Verschieben der Ausstosserhülse 17 nach vorne wird auch der Ausstosser 15 nach vorne geschoben und stösst den Zapfen aus.

Zum Sterilisieren lässt sich die Vorrichtung vom Gewinde 6a der Antriebswelle 6 abschrauben. Da diese angeschraubte Vorrichtung nur Metallteile hat, lässt sie sich ohne weiteres sterilisieren. Sie kann auch verhältnismässig leicht gereinigt werden: Nach dem Lösen der Imbus-Schraube 8 kann man den Fräser 9 dem Fräserhalter entnehnen, wodurch die Spannzange 10 freigelegt wird, sodass sich Fräser und Spannzange getrennt reinigen lassen. Falls ein Antrieb mit sterilisierbarem Elektromotor verwendet wird, kann das ganze Gerät ohne vorherige Zerlegung im Autoklav sterilisiert werden.

## Patentansprüche

1. Vorrichtung mit einem zum Herausschneiden eines Knorpel- oder Knochenzapfens dienenden, mit einer Antriebswelle (6) drehfest verbindbaren Hohlfräser (9), in dessen Innerem sich eine relativ zum Fräser längsverschiebbare, als Extraktor dienende Spannzange (10) befindet, dadurch gekennzeichnet, dass innerhalb dieser Spannzange ein längsverschiebbarer Ausstosser (15) angeordnet ist und dass der Hohlfräser (9) in einem hülsenförmigen, mit der Antriebswelle (6) fest verbundenen Fräserhalter (7) lösbar gehalten ist, welcher ein Aussengewinde (7e) aufweist, auf welchem eine vor- und rückwärts verschraubbare, mit einer Schiebehülse (13) versehene Rändelmutter (14) angeordnet ist, wobei die Schiebehülse (13) durch mindestens einen in einem Schlitz (7h) des Fräserhalters (7) zwischen zwei Endstellungen längs verschiebbaren, radialen Stiften (12) fest mit der Spannzange (10) verbunden ist.

2. Vorrichtung nach Anspruch 1, dadurch gekennzeichnet, dass die Aussenwandung der Spannzange (10) an der Innenwand der Bohrung des Hohlfräsers (9) anliegt, dass sich die Hohlfräserwandung gegen das freie Fräserende (9a) hin nach innen so verdickt, dass einerseits an der Fräsenmündung die lichte Weite des Fräsers der lichten Weite der Spannzange (10) in der zurückgezogenen Stellung entspricht, und andererseits die freien Enden (10a) der Spannzange sich beim nach-vorne-Verschieben einander nähern und dadurch die Grösse der Zangenöffnung verkleinern.

3. Vorrichtung nach Anspruch 1 oder Anspruch 2, dadurch gekennzeichnet, dass, falls der Hohlfräser (9) rechtsfräsend ist, das Aussengewinde (7e) auf dem Fräserhalter (7) ein Links-Gewinde ist.

4. Vorrichtung nach Anspruch 1, dadurch gekennzeichnet, dass der Ausstosser (15) durch einen axial verschiebbaren Stift gebildet wird, der mittels eines radialen, in einer Längsnut (7f) des Fräserhalters (7) zwischen zwei Endstellungen verschiebbaren Stiftes (16) fest mit einer Ausstosser-Hülse (17) verbunden ist, deren dem Fräser abgewandtes Ende (17a) auf dem Fräserhalter (7) aufliegt und die durch eine federnde Rast (7g/17c/18) in der zurückgezogenen Stellung festgehalten wird.

## Claims

1. Device with a tubular milling cutter (9), which serves for the excision of a cartilage or bone plug, is connectible rotationally fast with a drive shaft (6) and in the interior of which is disposed a grip chuck (10) serving as an extractor and longitudinally displaceable relative to the milling cutter, characterised in that a longitudinally displaceable ejector (15) is arranged within this grip chuck and that the tubular milling cutter (9) is releasably held in a sleeve-shaped milling cutter holder (7), which is fixedly connected with the drive shaft (6) and which has an external thread (7c) on which is arranged a forwardly and rearwardly screwable knurled nut (14) provided with a sliding sleeve (13), wherein the sliding sleeve (13) is fixedly connected with the grip chuck (10) by at least one radial pin (12) longitudinally displaceable in a slot (7h) of the milling cutter holder (7) between two end settings.

2. Device according to claim 1, characterised in that the outer wall of the grip chuck (10) bears against the inner wall of the bore of the tubular milling cutter (9), that the tubular milling cutter wall is so thickened inwardly towards the free end (9a) of the milling cutter that on the one hand the clear width of the milling cutter at the milling cutter mouth corresponds to the clear width of the grip chuck (10) in the retracted setting and on the other hand the free ends (10a) of the grip chuck approach each other during forward displacement and thereby reduce the size of the chuck opening.

3. Device according to claim 1 or 2, characterised in that in the case where the milling cutter (9) cuts in clockwise sense the external thread (7e) on the milling cutter holder is a left-handed thread.

4. Device according to claim 1, characterised thereby that the ejector (15) is formed by an axially displaceable pin, which is fixedly connected, by means of a pin (16) radially displaceable in a longitudinal groove (7f) of the milling cutter holder (7) between two end settings, with an ejector sleeve (17), the end (17a) of which remote from the milling cutter bears on the milling cutter holder (7) and which is held in the retracted setting by a resilient detent (7g/17c/18).

## Revendications

1. Dispositif comprenant une fraise creuse (9) susceptible d'être liée en rotation à un arbre d'entraînement (6), et destinée à prélever, par coupe, une carotte de cartilage ou d'os, fraise à l'intérieur de laquelle se trouve une pince de serrage (10) pouvant coulisser longitudinalement par rapport à la fraise et faisant office d'extracteur, caractérisé en ce qu'à l'intérieur de cette pince de serrage est disposé un éjecteur pouvant coulisser longitudinalement, et en ce que la fraise creuse (9) est maintenue de manière amovible dans un porte-fraise (7) en forme de douille, relié de manière fixe à l'arbre d'entraînement (6) et présentant un filetage extérieur (7e) sur lequel est monté un écrou moleté (14) pouvant être vissé vers l'avant et vers l'arrière, et pourvu d'une douille coulissante (13), cette douille coulissante (13) étant reliée de manière fixe à la pince de serrage (10), au moyen d'au moins une goupille radiale (12) pouvant coulisser longitudinalement entre deux positions extrêmes, dans une fente (7h) du porte-fraise (7).

2. Dispositif selon la revendication 1, caractérisé en ce que la paroi extérieure de la pince de serrage (10) est en appui sur la paroi intérieure de l'alésage de la fraise creuse (9), et en ce que l'épaisseur de la paroi de la fraise creuse augmente vers l'intérieur en direction de l'extrémité libre (9a) de la fraise, de manière telle, que d'une part, à l'orifice de la fraise, le diamètre intérieur de la fraise corresponde au diamètre intérieur de la pince de serrage (10) en position de retrait, et que d'autre part, les extrémités libres (10a) de la pince de serrage se rapprochent les unes des autres lors de leur coulissement vers l'avant, en réduisant ainsi la grandeur de l'ouverture de la pince.

3. Dispositif selon la revendication 1 ou la revendication 2, caractérisé en ce que dans le cas d'une fraise creuse (9) à fraisage à droite, le filetage extérieur (7e) sur le porte-fraise (7), est un filetage à gauche.

4. Dispositif selon la revendication 1, caractérisé en ce que l'éjecteur (15) est formé par une broche, qui peut coulisser dans la direction axiale, et qui, par l'intermédiaire d'une goupille radiale (16) pouvant coulisser dans une rainure longitudinale (7h) du porte-fraise (7), entre deux positions extrêmes, est reliée de manière fixe à une douille d'éjecteur (17) dont l'extrémité (17a) à l'opposé de celle dirigée vers la fraise, repose sur le porte-fraise (7), et est maintenue dans la position de retrait arrière, au moyen d'un système d'encliquetage élastique (7g/17c/18).
